# EUROPÄISCHE PATENTSCHRIFT

(11) **EP 0 955 023 B1**
(45) Veröffentlichungstag und Bekanntmachung des Hinweises auf die Patenterteilung: **25.06.2003**
(21) Anmeldenummer: 99108849.3
(22) Anmeldetag: 04.05.1999
(51) Int. Cl.: A61F 5/01, A61F 5/14

(54) **Orthese**
Orthesis
Orthèse

(30) Priorität: 08.05.1998 DE 29808341 U
(43) Veröffentlichungstag der Anmeldung: 10.11.1999
(73) Patentinhaber: Münch, Thomas, 47167 Duisburg (DE); Settner, Meinald Dr., 40885 Ratingen (DE)
(72) Erfinder: Münch, Thomas, 47167 Duisburg (DE); Settner, Meinald Dr., 40885 Ratingen (DE)
(74) Vertreter: Röther, Peter, Dipl.-Phys.

(56) Entgegenhaltungen:
- DE-U- 8 808 919
- DE-U- 9 115 983
- DE-U- 9 312 268
- DE-U- 9 420 046

## Beschreibung

Die Erfindung betrifft eine Orthese, insbesondere Fersenentlastungsorthese, bestehend aus einem zumindest den unteren Teil des Unterschenkels wenigstens teilweise umschließenden Schaft und einem über ein schalenförmig ausgebildetes Fersenteil mit dem Schaft verbundenes Fußteil, wobei zwischen der Spitze des Fußteils und dem Fersenteil eine die Orthese nach unten abschließende durchgehende Lauffläche angeordnet ist.

Derartige Orthesen werden eingesetzt beispielsweise nach Frakturen im Fersenbein- und Rückfußbereich, wobei es in erster Linie darauf ankommt, daß im Genesungsstadium das Fersenbein drucklos und ohne Berührung freischwebend positioniert ist. Die Erfindung geht aus von einer Orthese, wie sie in dem DE-GM 94 20 046.7 offenbart ist.

Durch die Ausgestaltung dieser bekannten Orthese wird eine dorsale Anlage des Schaftes im Wadenbereich und eine Unterstützung des Längsgewölbes erreicht, die zum einen eine freischwebende Positionierung des Fersenbeines in der Orthese gewährleistet, zum anderen durch die an einen modernen Skischuh erinnernde Ausgestaltung der Orthese ein fast normaler Abrollvorgang beim Gehen ermöglicht wird. Die Ferse dieser Orthese ist nach hinten ausgebaut und weist durch die schalenförmige Ausgestaltung eine Abrundung an der Hinterkante der Ferse auf. Dadurch werden die beim Aufsetzen des Fußes beim Bewegungsablauf auftretenden Kräfte vom Fersenteil in den an der Wade anliegenden Schaft geleitet. Beim weiteren Abrollvorgang wird das freischwebende Fersenbein nicht belastet, da die zu diesem Zeitpunkt auftretenden Kräfte über den Bogen in das Längsgewölbe des Fußes geleitet werden.

Durch die dorsale Anlage im Wadenbereich und die Unterstützung des Längsgewölbes, die dadurch erreicht wird, daß zwischen dem Absatz und dem Fußteil auf der Fußinnenseite ein Bogen ausgebildet ist, der von der vorderen Absatzkante in etwa rechtwinklig nach oben aufsteigt und seinen höchsten Punkt in etwa in Höhe des oberen Beginns der Fersenrundung besitzt und von dort die gegenüberliegende Flanke des Bogens flacher zum zehenseitigen Ende des Fußteils abfällt, ist eine feste Einschnürung der Unterschenkelmuskulatur nicht notwendig. Ebenso unnötig ist eine Schuhsohlenerhöhung am gesunden Fuß, da die Orthese den Fuß nur ca 1-2 cm höher positioniert als die gegenüberliegende Seite.

Trotz dieser offensichtlichen Vorteile weist die vorbekannte Orthese aufgrund ihrer speziellen Ausgestaltung die Tatsache auf, daß sie für jeden Fuß individuell angefertigt werden muß, da die Längsgewölbestütze in die Orthese integriert ist, die beispielsweise im Thermoformverfahren hergestellt wird, wobei jeweils andere Formwerkzeuge für verschiedene Größen und für den linken bzw. rechten Fuß benötigt werden. Hierdurch wird die Herstellung kostenintensiv, da die Formwerkzeuge teuer sind. Darüber hinaus bedingt die Ausgestaltung der vorbekannten Orthese eine unerwünschte Lagerhaltung für verschiedene Größen bzw. für den linken bzw. rechten Fuß.

Der Erfindung liegt daher die Aufgabe zugrunde, eine Orthese der eingangs genannten Art so auszubilden, daß zum einen die Herstellungskosten verringert werden und zum anderen eine Sortimentsreduzierung erreicht werden kann.

Die Erfindung löst diese Aufgabe gemäß dem kennzeichnenden Teil des Anspruchs 1 dadurch, daß die Lauffläche zumindest auf der ins Innere der Orthese weisenden Seite eben und symmetrisch zu ihrer Mittellängsachse ausgebildet ist und daß auf dieser ebenen Fläche eine Fußlängsgewölbestütze fixierbar ist, deren fußaußenseitiger Bereich in etwa horizontal verläuft, während der fußinnenseitige Bereich einen überhöhten bogenförmigen Verlauf aufweist, wobei die fersenseitige Flanke des Bogens in etwa senkrecht von der ebenen Lauffläche aufsteigt, in einen Scheitelbereich übergeht und von diesem in spitzem Winkel zum Fußteil bzw. zur Lauffläche verläuft.

Durch die symmetrische Ausgestaltung der Lauffläche und damit der gesamten Orthese wird erreicht, daß sie ohne weiteres sowohl für den rechten als auch für den linken Fuß geeignet ist. Weiterhin wird hierdurch erreicht, daß lediglich zwei Grundgrößen (Kinder und Erwachsene) benötigt werden, wobei zur spezielleren Größenanpassung lediglich ein Teil der Spitze der Orthese abgeschnitten werden muß.

Somit werden zur Herstellung der Orthese bzw. der Orthesenschale lediglich maximal zwei Formwerkzeuge benötigt, was die Herstellungskosten entscheidend reduziert.

Zur Anpassung an den verletzten Fuß muß nur die jeweilige Fußlängsgewölbestütze entsprechend angefertigt werden, die dann an entsprechender Stelle in der Orthesenschale auf die ebene Lauffläche aufgesetzt und dort fixiert werden muß.

Das geschieht entweder mittels eines geeigneten Klebers oder aber durch eine Haftreibungsverbindung, auch Klettverbindung genannt. Diese zweite Alternative hat den Vorteil, daß die Orthesenschale immer wieder verwendet werden kann und zum anderen, daß im Laufe des Genesungsprozesses die Fußlängsgewölbestütze ausgetauscht werden kann.

In einer bevorzugten Ausführungsform ist die Orthese als einstückiges Kunststoffteil ausgebildet, bei dem der Schaft im Wadenbereich beidseitig um den Unterschenkel herumgelegt und vorne verschlossen werden kann, ebenso wie das Fußteil, das vom Sohlenbereich um den Fuß herum im Spannbereich ebenfalls verschlossen werden kann.

Als Schnellverschluß eignen sich vorteilhafterweise Klettverschlüsse, die es ermöglichen, daß die Orthese sich dem Unterschenkel und Fußbereich in gewissen Toleranzen anpassen kann.

Zur Steigerung der Laufsicherheit wird vorgeschlagen, daß sowohl der Absatz als auch die Unterseite des Fußteils mit einer rutschfesten Laufsohle versehen sind.

Damit auch die Zehen geschützt sind, ist vorgesehen, daß das Fußteil eine die Zehen umschließende Kappe mit einer rutschfesten Sohle aufweist.

In einer weiteren vorteilhaften Ausführungsform sind im Bereich zwischen Schaft und Fersenteil beiderseitig der Orthese Knöchelschutzpolster vorgesehen.

Sowohl zu Kontrollzwecken als auch zur besseren Entlüftung der schalenförmig ausgebildeten Orthese ist hinten im Fersenteil oberhalb des Absatzes eine Öffnung vorgesehen.

Die Erfindung wird im folgenden anhand von Zeichnungen dargestellt und näher erläutert.

Es zeigen:
- Fig. 1:: eine Orthese von der Fußinnenseite gesehen,
- Fig. 2:: eine Orthese von der Fußaußenseite gesehen,
- Fig. 3:: Fußlängsgewölbestütze in Seitenansicht (Fußinnenseite),
- Fig. 4:: Fußlängsgewölbestütze in Vorderansicht.

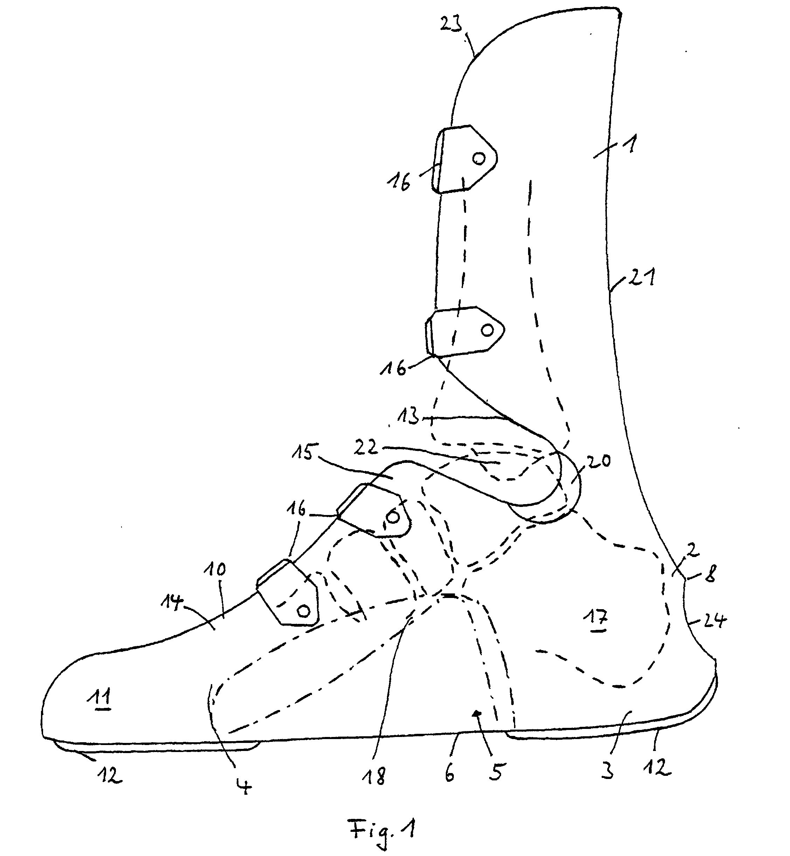

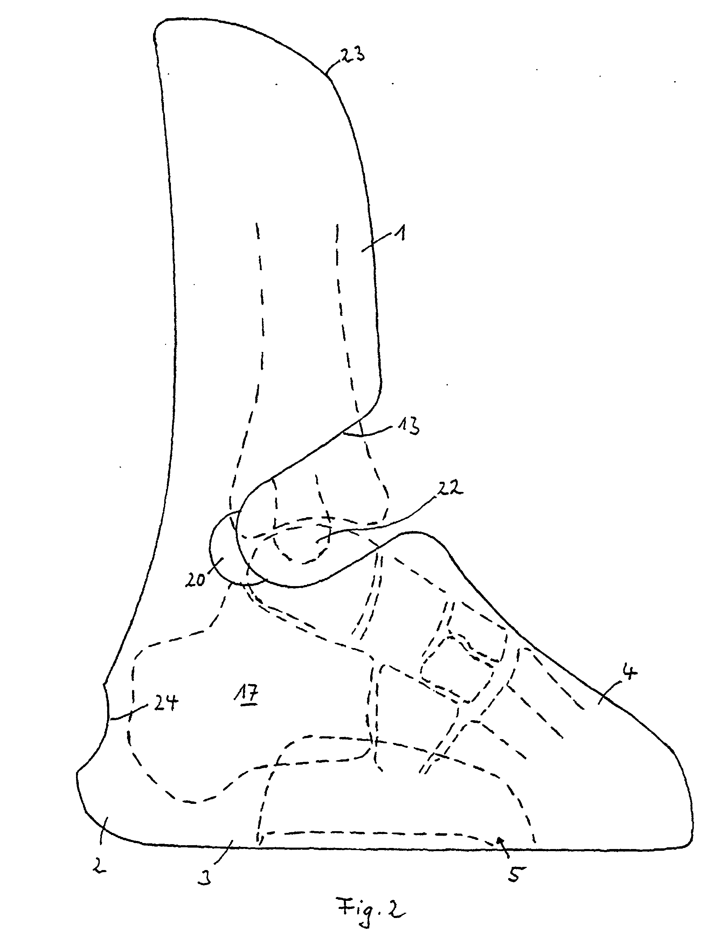

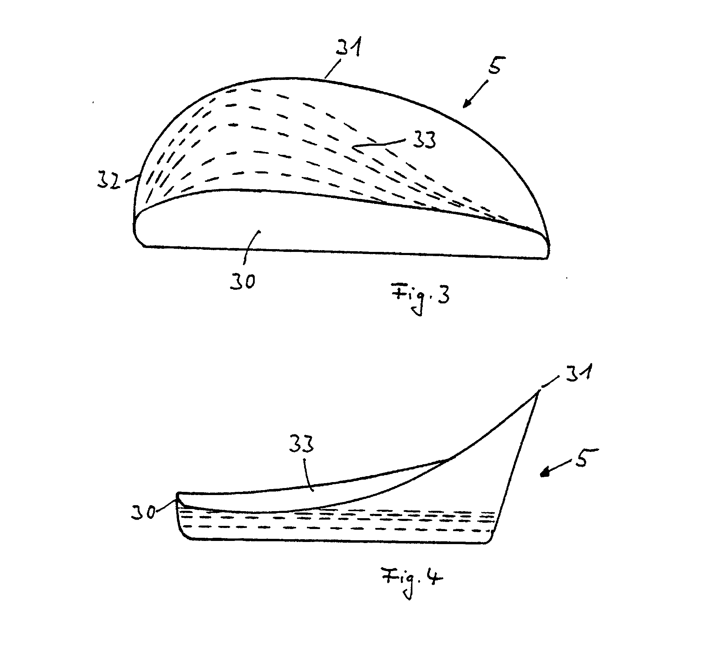

Das in den Fig. 1 und 2 dargestellte Ausführungsbeispiel einer erfindungsgemäßen Orthese weist einen Schaft 1, ein sich an diesen Schaft 1 anschließendes Fersenteil 2 und ein vom Fersenteil 2 ausgehendes Fußteil 4 auf. Zwischen Absatz 3 und Fußteil 4 ist in der Orthese eine Fußlängsgewölbestütze 5 angeordnet, die weiter unten im einzelnen beschrieben wird.

An das vorne offene Ende des Fußteils 4 ist eine die Zehen umschließende Kappe 11 angeformt.

Sowohl unter dem Fersenteil 2 als auch unter der Kappe 11 ist eine rutschfeste Laufsohle 12 angeordnet.

Schaft 1, Fersenteil 2 und Fußteil 4 bilden eine Einheit und sind vorteilhafterweise aus einem geeigneten Kunststoff beispielsweise im Thermoformverfahren hergestellt.

Der Schaft 1 reicht im hinteren Beinbereich bis in etwa Wadenmitte, während an den Beinseiten die Oberkante des Schaftes 1 einen konvexen Verlauf 21 bis in den Schienbeinbereich aufweist. In Höhe der Malleolengabel 22 tritt die Randkontur des Schaftes 1 bis in die Nähe der Knöchel nach hinten zurück, um jeweils eine Ausbuchtung 13 zu bilden, die den Malleolengabelbereich ausspart, um so diesen Bereich druckfrei zu halten.

Von den Ausbuchtungen 13 verlaufen die Randkonturen des Fußteils 4 wieder aufeinander zu, wobei sie sich am Kappenrand 14 treffen.

Die Orthese ist so ausgestaltet, daß die den Fußteil mit dem Fersenteil verbindende Lauffläche auf ihrer in die Orthese hineinweisenden Seite eben ausgebildet ist und diese Grundfläche symmetrisch zur Mittellängsachse ist. Auf diese ebene Lauffläche 6 ist eine Fußlängsgewölbestütze 5 aufgesetzt, beispielsweise geklebt. Wie aus den Fig. 3 und 4 hervorgeht, handelt es sich um ein separates Formteil mit zwei unterschiedlich hohen, schräg aufsteigenden Seitenwänden 30, 31, wobei die Fläche 33 dazwischen dem orthopädischen Zweck entsprechend ausgeformt ist, derart, daß die fußaußenseitige Flächenbegrenzung 30 in etwa horizontal verlaufend ausgebildet ist, die fußinnenseitige Flächenbegrenzung 31 aber in Form eines Bogens verläuft, wobei die fersenseitige Flanke 32 des Bogens in etwa rechwinklig bis zum Scheitelpunkt des Bogens ansteigt und von dort in flacherem Winkel abfällt und an ihrem Ende in etwa die gleiche Höhe erreicht wie die fußaußenseitige Flächenbegrenzung.

Nachdem der Patient von oben in das aufklaffende Fußteil 4 und mit den Zehen in die Kappe 11 geschlüpft ist, werden die Seitenteile 14 und 15 des Fußteils 4 aufeinander zu um den Fuß gelegt und mittels zweier Haftverschlüsse 16 miteinander verbunden. Der Scheitelbereich des fußinnseitigen Bogens der Fußlängsgewölbestütze liegt nun vor dem im Fersenteil 2 freischwebend positionierten Fersenbein 17 und unterstützt im Zusammenwirken mit der flacher abfallenden Flanke des Bogens sowohl das Längsgewölbe 18 des lediglich angedeuteten Fußes als auch die Malleolengabel 22.

Danach wird der ebenfalls zunächst vorne aufklaffende Schaft 1 beidseitig um den Unterschenkel gelegt und im Schienbeinbereich ebenfalls mittels zweier Haftverschlüsse 16 geschlossen. Im Bereich zwischen Schaft 1 und Fersenteil 2, angrenzend an die Ausbuchtung 13, ist die Orthese mit Knöchelschutzpolstern 20 versehen.

So präpariert kann der zu behandelnde Fuß beim Bewegungsablauf einen praktisch normalen Abrollvorgang ausführen, da beim Aufsetzen des Fußes, also am Beginn des Abrollvorganges, der abgerundete Fersenteil 2 die Kräfte aufnimmt und in den Wadenbereich 21 des Schaftes weiterleitet.

Im weiteren Verlauf des Abrollvorganges übernimmt die Fußlängsgewölbestütze 5 die Kraftaufnahme. Während des gesamten Vorgangs ist das Fersenbein vollständig entlastet.

Zu Kontrollzwecken und zur besseren Durchlüftung der im angelegten Zustand in etwa skischuhförmig ausgebildeten Orthese ist zumindest im Fersenbereich oberhalb des Absatzes 3 eine Öffnung 24 vorgesehen. Eine derartige Öffnung kann auch in der Kappe 11 vorgesehen sein.

## Patentansprüche

1. Orthese, insbesondere Fersenentlastungsorthese, bestehend aus einem zumindest den unteren Teil des Unterschenkels wenigstens teilweise umschließenden Schaft und einem über ein schalenförmig ausgebildetes Fersenteil mit dem Schaft verbundenes Fußteil, wobei zwischen der Spitze des Fußteils und dem Fersenteil eine die Orthese nach unten abschließende durchgehende Lauffläche angeordnet ist,
**dadurch gekennzeichnet,**
**daß** die Lauffläche (6) zumindest auf der ins Innere der Orthese weisenden Seite eben und symmetrisch zu ihrer Mittellängsachse ausgebildet ist und daß auf dieser ebenen Fläche (6) eine Fußlängsgewölbestütze (5) fixierbar ist, deren fußaußenseitiger Bereich (30) in etwa horizontal verläuft, während der fußinnenseitige Bereich (31) einen überhöhten bogenförmigen Verlauf aufweist, wobei die fersenseitige Flanke des Bogens in etwa senkrecht von der ebenen Lauffläche (6) aufsteigt, in einen Scheitelbereich übergeht und von diesem in spitzem Winkel zum Fußteil (4) verläuft.

2. Orthese nach Anspruch 1,
**dadurch gekennzeichnet,**
**daß** die Fußlängsgewölbestütze (5) mittels eines Klebers fixierbar ist.

3. Orthese nach Anspruch 1,
**dadurch gekennzeichnet,**
**daß** die Fußlängsgewölbestütze (5) mittels einer Haftreibungsverbindung fixierbar ist.

4. Orthese nach einem der Ansprüche 1 bis 3,
**dadurch gekennzeichnet,**
**daß** die Fußlängsgewölbestütze (5) aus einem Kunststoff geformt ist.

5. Orthese nach Anspruch 4,
**dadurch gekennzeichnet,**
**daß** die Fußlängsgewölbestütze (5) aus einem Schaumkunststoff geformt ist.

6. Orthese nach einem der Ansprüche 1 bis 5,
**dadurch gekennzeichnet,**
**daß** Schaft (1), Fersenteil (2) und Fußteil (4) als einstückiges Kunststoffteil ausgebildet sind.

7. Orthese nach einem der Ansprüche 1 bis 6,
**dadurch gekennzeichnet,**
**daß** Schaft (1) und Fußteil (4) mit Haftverschlüssen (16) versehen sind, die im Schienbeinbereich bzw. im Spannbereich eine lösbare Verbindung bilden.

8. Orthese nach einem der Ansprüche 1 bis 7,
**dadurch gekennzeichnet,**
**daß** sowohl unter dem Fersenteil als auch unter dem Fußteil eine rutschfeste Sohle (12) vorgesehen ist.

9. Orthese nach einem der Ansprüche 1 bis 8,
**dadurch gekennzeichnet,**
**daß** das Fußteil (4) eine die Zehen umschließende Kappe (11) mit einer rutschfesten Sohle (12) aufweist.

10. Orthese nach einem der Ansprüche 1 bis 9,
**dadurch gekennzeichnet,**
**daß** im Bereich zwischen Schaft (1) und Fersenteil (2) beiderseits Knöchelschutzpolster (20) vorgesehen sind.

11. Orthese nach einem der Ansprüche 1 bis 10,
**dadurch gekennzeichnet,**
**daß** im Fersenteil (2) oberhalb der Lauffläche eine Öffnung (24) vorgesehen ist.

## Claims

1. An orthesis, in particular a heel-relieving orthesis, consisting of a shaft at least partially surrounding at least the lower part of the lower leg, and a foot part connected to the shaft via a heel part of a dish-shaped design, wherein a continuous walking surface, closing off the orthesis towards the bottom, is arranged between the tip of the foot part and the heel part,
**characterised in that**
the walking surface (6) is designed in such a way as to be flat and symmetrical about its centre-line axis at least on the side facing the inside of the orthesis, and a plantar arch support (5) is fixable on this flat surface (6), the region (30) of which on the outside of the foot follows a roughly horizontal course whereas the region (31) on the inside of the foot exhibits a high-pitched arcuate course, the flank of the arc on the heel side rising roughly perpendicularly from the flat walking surface (6) and undergoing a transition into an apical region from which it follows a course at an acute angle towards the foot part (4).

2. An orthesis according to Claim 1,
**characterised in that**
the plantar arch support (5) is fixable with the use of a bonding agent.

3. An orthesis according to Claim 1,
**characterised in that**
the plantar arch support (5) is fixable with the use of a static-friction composition.

4. An orthesis according to one of Claims 1 to 3,
**characterised in that**
the plantar arch support (5) is formed from a plastics material.

5. An orthesis according to Claim 4,
**characterised in that**
the plantar arch support (5) is formed from a foamed plastics material.

6. An orthesis according to one of Claims 1 to 5,
**characterised in that**
the shaft (1), heel part (2) and foot part (4) are in the form of an integral plastics-material part.

7. An orthesis according to one of Claims 1 to 6,
**characterised in that**
the shaft (1) and foot part (4) are provided with hook-and-loop fasteners (16), which form a releasable connection in the shin region and/or in the instep region.

8. An orthesis according to one of Claims 1 to 7,
**characterised in that**
a non-slip sole (12) is provided both beneath the heel part and beneath the foot part.

9. An orthesis according to one of Claims 1 to 8,
**characterised in that**
the foot part (4) has a toe-enclosing cap (11) with a non-slip sole (12).

10. An orthesis according to one of Claims 1 to 9,
**characterised in that**
ankle-protection cushions (20) are provided on both sides in the region between the shaft (1) and the heel part (2).

11. An orthesis according to one of Claims 1 to 10,
**characterised in that**
an opening (24) is provided in the heel part (2) above the walking surface.

## Revendications

1. Orthèse, en particulier orthèse de décharge du talon, comprenant une tige entourant au moins la partie inférieure de la jambe, au moins partiellement, et un élément de pied relié à la tige par un élément de talon en forme de coque, une surface de marche continue fermant l'orthèse vers le bas étant disposée entre la pointe de l'élément de pied et l'élément de talon, **caractérisée en ce que**
la surface de marche (6) est formée au moins sur le côté tourné vers l'intérieur de l'orthèse de façon plane et symétrique à son axe longitudinal central, et **en ce que** sur cette surface plane (6) peut être fixé un soutien de la voûte longitudinale (5), dont la zone située sur le côté latéral extérieur du pied (30) se situe sur un plan à peu près horizontal, tandis que la zone située sur le côté latéral intérieur du pied (31) présente un profil en forme d'arc surélevé, le flanc de l'arc situé du côté de talon s'élevant selon une direction à peu près perpendiculaire par rapport à la surface de marche (6) plane, puis évoluant en une zone sommitale, puis s'étendant depuis cette zone vers l'élément de pied (4) selon un angle aigu.

2. Orthèse selon la revendication 1, **caractérisée en ce que** le soutien de la voûte longitudinale (5) peut être fixé au moyen d'une colle.

3. Orthèse selon la revendication 1, **caractérisée en ce que** le soutien de la voûte longitudinale (5) peut être fixé au moyen d'une liaison par frottement par adhérence.

4. Orthèse selon l'une des revendications 1 à 3, **caractérisée en ce que** le soutien de la voûte longitudinale (5) est moulé en une matière plastique.

5. Orthèse selon la revendication 4, **caractérisée en ce que** le soutien de la voûte longitudinale (5) est moulé en une matière plastique cellulaire.

6. Orthèse selon l'une des revendications 1 à 5, **caractérisée en ce que** la tige (1), l'élément de talon (2) et l'élément de pied (4) sont constitués sous la forme d'un élément de matière plastique d'une seule pièce.

7. Orthèse selon l'une des revendications 1 à 6, **caractérisée en ce que** la tige (1) et l'élément de pied (4) sont pourvus de fermetures adhésives (16), qui forment une liaison amovible dans la zone du tibia et respectivement du cou-de-pied.

8. Orthèse selon l'une des revendications 1 à 7, **caractérisée en ce qu'**une semelle antidérapante (12) est prévue tant sous l'élément de talon que sous l'élément de pied.

9. Orthèse selon l'une des revendications 1 à 8, **caractérisée en ce que** l'élément de pied (4) présente un bout (11) englobant les orteils avec une semelle antidérapante (12).

10. Orthèse selon l'une des revendications 1 à 9, **caractérisée en ce que** des coussins de protection de la cheville (20) sont prévus des deux côtés dans la zone comprise entre la tige (1) et l'élément de talon (2).

11. Orthèse selon l'une des revendications 1 à 10, **caractérisée en ce qu'**une ouverture (24) est prévue dans l'élément de talon (2) au-dessus de la surface de marche.
